# EUROPEAN PATENT SPECIFICATION

(11) **EP 1 875 906 B1**
(45) Date of publication and mention of the grant of the patent: **29.06.2011**
(21) Application number: 07017630.0
(22) Date of filing: 21.07.2004
(51) Int. Cl.: A61K 31/35, C12Q 1/44

(54) **Therapeutic use of yessotoxin as human tumor cell growth inhibitor**
Therapeutische Verwendung von Yessotoxin als Wachstumshemmer für humane Tumorzellen
Utilisation thérapeutique de yessotaxine en tant qu'inhibiteur de croissance de cellule de tumeur humaine

(30) Priority: 25.07.2003 ES 200301773
(43) Date of publication of application: 09.01.2008
(62) Divisional of application: 04742071.6
(73) Proprietor: LABORATORIO CIFGA, S.A., 27001 Lugo (ES)
(72) Inventor: Botana López, Luis Miguel, Santiago de Compostela A Coruña (ES); Alfonso Rancaño, Amparo, Santiago de Compostela A Coruña (ES); Rodríguez Vieytes, Mercedes, Santiago de Compostela A Coruña (ES); Loza García, María Isabel, Santiago de Compostela A Coruña (ES)
(74) Representative: ABG Patentes, S.L.

(56) References cited:
- WO-A1-99/35283
- US-A- 6 156 528
- MALAGUTI C ET AL: "Caspase activation and death induced by yessotoxin in HeLa cells." TOXICOLOGY IN VITRO : AN INTERNATIONAL JOURNAL PUBLISHED IN ASSOCIATION WITH BIBRA AUG 2002, vol. 16, no. 4, August 2002 (2002-08), pages 357-363, XP002464327 ISSN: 0887-2333
- LEIRA F ET AL: "Characterization of distinct apoptotic changes induced by okadaic acid and yessotoxin in the BE(2)-M17 neuroblastoma cell line" TOXICOLOGY IN VITRO, ELSEVIER SCIENCE, GB, vol. 16, no. 1, February 2002 (2002-02), pages 23-31, XP002995442 ISSN: 0887-2333
- ALFONSO A ET AL: "Yessotoxin, a novel phycotoxin, activates phosphodiesterase activity. Effect of yessotoxin on cAMP levels in human lymphocytes" BIOCHEMICAL PHARMACOLOGY, PERGAMON, OXFORD, GB, vol. 65, no. 2, 15 January 2003 (2003-01-15), pages 193-208, XP002995441 ISSN: 0006-2952
- HIRSH LIRON ET AL: "Phosphodiesterase inhibitors as anti-cancer drugs.", BIOCHEMICAL PHARMACOLOGY 15 SEP 2004 LNKD- PUBMED:15313391, vol. 68, no. 6, 15 September 2004 (2004-09-15), pages 981-988, ISSN: 0006-2952
- NICOLAUS B J R: "SYMBIOTIC APPROACH TO DRUG DESIGN", DECISION MAKING IN DRUG RESEARCH, XX, XX, 1 January 1983 (1983-01-01), pages 173-186, XP001111439,

## Description

The present invention relates to the therapeutic use of yessotoxin as a cytotoxic agent for human hepatocellular carcinoma cells due to its ability to activate cellular phosphodiesterases.

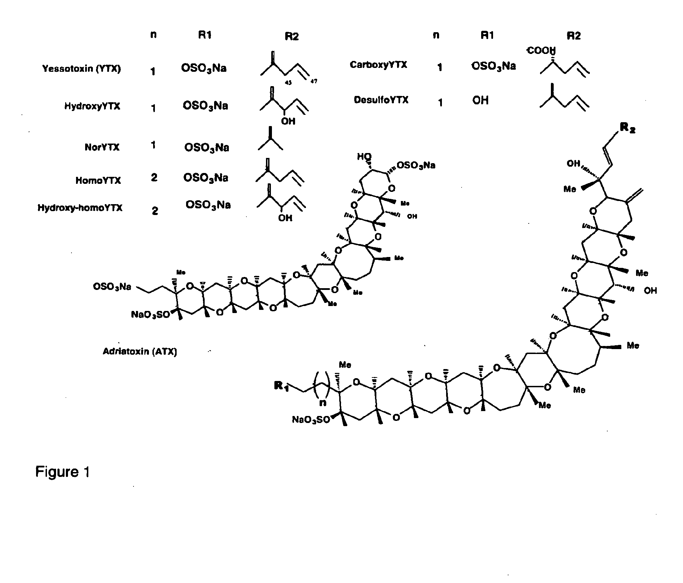

Yessotoxin, hereinafter YTX, and its natural analogs are polycyclic ethers produced by dinoflagellates of the *Protoceratium reticulatum* and *Lingolodinium polyedrum* species and originally isolated from the digestive apparatus of *Patinopecten yessoensis* (Murata, M., Kumagai, M. et al., 1987, Tetrahedron Letters, 28, 5869-5872). Its molecule, represented in Figure 1, is formed by eleven rings with ether groups and an unsaturated side chain having different radicals bound thereto. This figure shows some YTX analogs, though there are over 50 natural derivative analogs.

YTX is a lipophilic compound that is not toxic when ingested orally (Aune, T., Sorby, R. et al., 2002, Toxicon, 40, 77-82); however, it causes death after intraperitoneal injection (Tubaro, A., Sosa, S. et al., 2003, Toxicon, 41, 783-92), although a very high dose is required.

YTXs and their analogs have a mechanism of action that is different from that of any other toxin. They were originally classified within the group of diarrhetic toxins, since they are often detected together; however, they are different in that they do not produce diarrhea, nor do they affect cellular phosphatases, the cellular target of diarrhetic toxins. Their mechanism of action is related to other enzymes; in this sense it has been described that YTX decreases adenosine cyclic phosphate (cAMP) cytosolic levels because it increases the activity of cellular phosphodiesterases by a calcium-dependent mechanism (Alfonso, A., de la Rosa, L.A. et al., 2003, Biochem Pharmacol, 65, 193-208). YTX further increases cytosolic calcium levels by stimulating its entry through a channel located in the cell membrane (De la Rosa, L.A., Alfonso, A. et al., 2001, Biochem. Pharmacol., 61, 827-833; De la Rosa, L.A., Alfonso, A. et al., 2001, Cell Signal, 13, 711-716). The effect on phosphodiesterases has been used to develop sensitive methods for detecting the presence of these toxins in contaminated mollusks, which have recently been published (Alfonso, A., Vieytes, M.R. et al., 2004, Analytical Biochem., 326, 93-99; Pazos, M.J., Alfonso, A. et al., 2004, Analytical Biochem., 335, 112-118). There are eleven families of phosphodiesterases which are very important from the pharmacological point of view given that their modulation is involved in the treatment of diseases such as asthma, rheumatoid arthritis and cancer (Houslay, M.D. and Adams, D.R., 2003, Biochem J., 370, 1-18).

YTX induces apoptosis, programmed cell death, in human neuroblastoma cells and in human cervix carcinoma cells (HeLa line) due to caspase activation (Leira, F., Alvarez, C. et al., 2001, Toxicology in vitro, 15, 277-283; Malaguti, C., Ciminello, P. et al., 2002, Toxicol in Vitro, 16, 357-363). This toxin further has a cytotoxic effect on human hepatocellular carcinoma cells (HEP-G2) and HeLa229 cells; therefore *a priori* it is susceptible to being used as an antitumor drug.

Due to both the levels of this second messenger and due to the activation taking place on the protein kinase A, the cAMP pathway is involved in cell proliferation. Protein kinase A inhibition gives rise to antitumor activity (Wang, H., Cai, Q. et al., 1999, Proc Natl Acad Sci U S A, 96, 13989-94), though it has also been described that the resistance to antitumor drugs, such as cisplatin, is related to the inactivation of this protein (Cvijic, M.E., Yang, W.L. et al., 1998, Pharmacol Ther, 78, 115-28). Incidentally, cAMP levels are in turn related to cytotoxicity and resistance to drugs (Mann, S.C., Andrews, P.A. et al., 1991, Int J Cancer, 48, 866-72; von Knethen, A., Lotero, A. et al., 1998, Oncogene, 17, 387-94). In other words, the cAMP pathway plays an important and complex role in regulating cell growth. For this reason, the description of natural or synthetic molecules affecting phosphodiesterases, and of methods for studying the activity of these enzymes, which can be applied in HTS (high throughput screening) protocols, are very useful tools for discovering new treatments. In this sense, the description of the inhibiting effect of YTXs on tumor cell growth is an indication of the pharmacological importance of these molecules for their possible therapeutic application. Furthermore, due to their low toxicity, some authors point out that YTX should not be considered a toxin but rather a natural product with different pharmacological uses.

These tests are used in the pharmaceutical industry to quickly evaluate the therapeutic value of natural products and of synthetic compound libraries. They are routine assays on cells or tissue in which the affinity, selectivity and specificity for the primary target and the action mechanism or the pharmaceutical properties of the compounds are determined. Those that fail in any parameter are thus discarded for subsequent studies

A more in-depth explanation of the families of toxins, the particular characteristics of YTXs and their mechanism of action is provided in the description of the main and divisional applications related to the present invention.

The proposed invention describes a use of YTX as a cell growth inhibitor of human hepatocellular carcinoma cells according to their ability to activate cellular phosphodiesterases.

### USE: Use of phosphodiesterases-activating YTX and compounds as neoplasic cell proliferation inhibitors.

Neoplasic cell growth inhibition is an indicator of antitumor activity widely used to describe antineoplasic properties of new drugs. It has been found that YTX is cytotoxic for human hepatocellular carcinoma cells, and it has further been described that this toxin induces apoptosis in neuroblastoma cells (Leira, F., Alvarez, C. et al., 2001, Toxicology in vitro, 15, 277-283), which all indicates that YTX is susceptible to being used as an antitumor drug. The ability of YTX as a cytotoxic drug for hepatic carcinoma tumor cells is quantified in the present use. Cell growth inhibition can be determined according to different protocols described in the literature. One of these protocols is set forth below in which the response is quantified in the HEP-G2 cell line by means of staining with crystal violet and subsequent acetylation.

### EMBODIMENT OF THE INVENTION

a.- HEP-G2 cells are seeded on a microtitration plate with a density of 10000 cells per well. They are incubated for 24 hours with growth medium at 37°C and 5% CO₂.
b.- Different concentrations of YTX are added and it is incubated for 48 hours at 37°C and 5% CO₂.
c.- 10 µL of 11% glutaraldehyde are added to fix the cells and it is incubated for 15 minutes. It is washed 3-4 times with distilled water.
d.- A 0.1 % solution of crystal violet is added and the plate is shaken for 15 minutes.
e.- The dye is removed by washing with distilled water and it is subsequently dried.
f.- 10% acetic acid is added and shaking is maintained for 15 minutes.
g.- Absorbance is read in a spectrophotometer at 595 nanometers.
h.- It was found with this protocol that 10 µM of YTX induce cell growth inhibition of about 82+/-1 %.

### LITERATURE

Alfonso, A., de la Rosa, L. A., Vieytes, M. R., Yasumoto, T. and Botana, L. M. (2003). "Yessotoxin a novel phycotoxin, activates phosphodiesterase activity. Effect of yessotoxin on cAMP levels in human lymphocytes." Biochem Pharmacol 65: 193-208.

Alfonso, A., Vieytes, M. R., Yasumoto, T. and Botana, L. M. (2004). "A rapid microplate fluorescent method to detect yessotoxins based on their capacity to activate phosphodiesterases." Analytical Biochem. 326: 93-99.

Aune, T., Sorby, R., Yasumoto, T., Ramstad, H. and Landsverk, T. (2002). "Comparison of oral and intraperitoneal toxicity of yessotoxin towards mice." Toxicon 40(1): 77-82.

Cvijic, M. E., Yang, W. L. and Chin, K. V. (1998). "Cisplatin resistance in cyclic AMP-dependent protein kinase mutants." Pharmacol Ther 78(2): 115-28.

De la Rosa, L. A., Alfonso, A., Vilariño, N., Vieytes, M. R. and Botana, L. M. (2001). "Modulation of cytosolic calcium levels of human lymphocytes by yessotoxin, a novel marine phycotoxin." Biochem. Pharmacol. 61 (7): 827-833.

De la Rosa, L. A., Alfonso, A., Vilariño, N., Vieytes, M. R., Yasumoto, T. and Botana, L. M. (2001). "Maitotoxin-induced calcium entry in human lymphocytes - Modulation by yessotoxin, Ca2+ channel blockers and kinases." Cell Signal 13(10): 711-716.

Houslay, M. D. and Adams, D. R. (2003). "PDE4 cAMP phosphodiesterases: modular enzymes that orchestrate signalling cross-talk, desentization and compartmentalization." Biochem J. 370: 1-18.

Leira, F., Alvarez, C., Vieites, J. M., Vieytes, M. R. and Botana, L. M. (2001). "Okadaic acid and yessotoxin induce caspase-3 mediated apoptosis in neuroblastoma cells. Characterization of distinct apoptotic changes induced by these phycotoxins in the BE(2)-M17 cell line by means of new fluorimetric microplate assays." Toxicology in vitro 15: 277-283.

Malaguti, C., Ciminello, P., Fattorusso, E. and Rossini, G. P. (2002). "Caspase activation and death induced by yessotoxin in HeLa cells." Toxicol in Vitro 16(4): 357-363.

Mann, S. C., Andrews, P. A. and Howell, S. B. (1991). "Modulation of cis-diamminedichloroplatinum(II) accumulation and sensitivity by forskolin and 3-isobutyl-1-methylxanthine in sensitive and resistant human ovarian carcinoma cells." Int J Cancer 48(6): 866-72.

Murata, M., Kumagai, M., Lee, J. S. and Yasumoto, T. (1987). "Isolation and structure of Yessotoxin, a novel polyether compound implicated in diarrhetic shellfish poisoning." Tetrahedron Letters 28: 5869-5872.

Pazos, M. J., Alfonso, A., Vieytes, M. R., Yasumoto, T., Vieites, J. M. and Botana, L. M. (2004). "Resonant mirror biosensor detection method based on yessotoxin-phosphodiesterase interactions." Analytical Biochem. 335: 112-118.

Tubaro, A., Sosa, S., Carbonatto, M., Altinier, G., Vita, F., Melato, M., Satake, M. and Yasumoto, T. (2003). "Oral and intraperitoneal acute toxicity studies of yessotoxin and homoyessotoxins in mice." Toxicon 41 (7): 783-92.

von Knethen, A., Lotero, A. and Brune, B. (1998). "Etoposide and cisplatin induced apoptosis in activated RAW 264.7 macrophages is attenuated by cAMP-induced gene expression." Oncogene 17(3): 387-94.

Wang, H., Cai, Q., Zeng, X., Yu, D., Agrawal, S. and Zhang, R. (1999). "Antitumor activity and pharmacokinetics of a mixed-backbone antisense oligonucleotide targeted to the Rlalpha subunit of protein kinase A after oral administration." Proc Natl Acad Sci U S A 96(24): 13989-94.

## Claims

1. Yessotoxin capable of activating cellular phosphodiesterases for use in the treatment of human hepatocellular carcinoma.

## Patentansprüche

1. Yessotoxin, das in der Lage ist, zelluläre Phosphodiesterasen zu aktivieren, zur Verwendung in der Behandlung von humanem Leberzellkarzinom.

## Revendications

1. Yessotoxine apte à activer des phosphodiesteroses cellulaires pour une utilisation dans le traitement du carcinome hépatocellulaire humain.
